Europäisches Patentamt

**European Patent Office**

**Office européen des brevets**

(11) Veröffentlichungsnummer: **0 019 929**
**A2**

(12) **EUROPÄISCHE PATENTANMELDUNG**

(21) Anmeldenummer: **80103041.2**

(22) Anmeldetag: **31.05.80**

(51) Int. Cl.³: **A 61 K 9/20,** A 61 K 9/44

(30) Priorität: **01.06.79 DE 2922522**

(43) Veröffentlichungstag der Anmeldung: **10.12.80**
**Patentblatt 80/25**

(84) Benannte Vertragsstaaten: **CH DE FR GB IT LI SE**

(71) Anmelder: **Dudzik, Joachim, Vor dem Klingler 1,**
**D-7440 Nürtingen (DE)**
Anmelder: **Dudzik, Winfried, Drosselweg 13,**
**D-7441 Neckartailfingen (DE)**

(72) Erfinder: **Dudzik, Joachim, Vor dem Klingler 1,**
**D-7440 Nürtingen (DE)**
Erfinder: **Dudzik, Winfried, Drosselweg 13,**
**D-7441 Neckartailfingen (DE)**

(74) Vertreter: **Maier, Eugen, Dr.-Ing. et al, Patentanwälte**
**Dr.-Ing. Eugen Maier Dr.-Ing. Eckhard Wolf**
**Pischekstrasse 19, D-7000 Stuttgart 1 (DE)**

(54) **Tablette und Verfahren zu deren Herstellung.**

(57) Bei einer Tablette mit einem Trägermaterial und mindestens einem im Trägermaterial enthaltenen Wirkstoff, insbesondere einem Nahrungsmittel, einem pharmazeutischen oder chemischen Präparat, Pflanzen- oder Tiersamen, ist das Trägermaterial (8) als saugfähiges, ein unter Wasseraufnahme aufquellendes Bindemittel enthaltendes Faservlies ausgebildet, das beim Aufquellen des Bindemittels selbsttätig in einen Faserbrei zerfällt. Der Wirkstoff (2) kann z.B. mit einer Mikrodosierpumpe in Form einer Lösung, Suspension oder Schmelze in das Faservlies injiziert oder auf dieses aufgetragen oder aufgedruckt werden. Das Trägermaterial besteht bevorzugt aus einem Zellstoffaservlies, dessen Fasern im trockenen Zustand durch ein hydrophiles Gel, z.B. durch Gelatine oder Knochenleim untereinander zusammengehalten werden. Das Faservlies kann mit einem porenverschließenden wasserlöslichen oder wasserdurchlässigen Überzug versehen sein, der zweckmäßig eine wasserabstoßende Oberfläche aufweist. Die neue Tablettenform zeichnet sich vor allem durch eine rationelle und billige Fertigung aus.

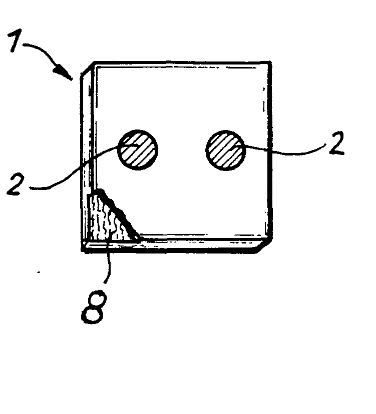

ACTORUM AG

BEZEICHNUNG GEÄNDERT
siehe Titelseite

## Beschreibung

Die Erfindung betrifft eine Tablette mit einem Trägermaterial und mindestens einem im Trägermaterial enthaltenen Wirkstoff, insbesondere einem Nahrungsmittel, einem pharmazeutischen oder chemischen Präparat oder Pflanzen- oder Tiersamen.

Unter einer Tablette soll in den Ansprüchen und in der folgenden Beschreibung im weitesten Sinne eine in fester Form vorliegende Portions- oder Einzeldosisverpackung für die Wirkstoffe verstanden werden, die bei der Anwendung in bestimmten Flüssigkeiten, beispielsweise in Wasser oder in einer Körperflüssigkeit unter Freigabe des Wirkstoffes aufgelöst wird.

In der Pharmazie werden bei der Tablettenherstellung üblicherweise die in Pulverform vorliegenden Wirkstoffe gegebenenfalls mit Füll-, Binde- und Auflockerungs- oder Gleitmitteln, wie Milchzucker, Stärke, Talk oder Kakaobutterlösung als Trägermaterial gemischt und nach einer eventuellen Granulierung zu Täfelchen, Zylindern oder Kugeln gepreßt und erforderlichenfalls mit einem Überzug aus Zucker, Kakao oder anderen Stoffen versehen. Hierfür gibt es zahlreiche Maschinen, die meist recht kompliziert aufgebaut sind und viel Raum beanspruchen. Der Einsatz derartiger Tablettiermaschinen erfordert einen hohen Kapital- und Personalaufwand, der sich unmittelbar auf die Herstellungskosten der Tabletten auswirkt. Hinzu kommt, daß vor allem bei niedriger Wirkstoffdosierung und bei schwerlöslichen Präparaten die Herstellung einer gleichmäßigen Mischung mit dem Trägermaterial und damit die Einhaltung einer ausreichenden Dosiergenauigkeit in den einzelnen Tabletten erhebliche Schwierigkeiten bereitet, die zum Teil bis heute noch nicht überwunden sind.

Der Erfindung liegt die Aufgabe zugrunde, eine Tablettenform der eingangs angegebenen Art sowie ein Verfahren zu deren Herstellung zu entwickeln, die eine rationelle und billige Fertigung ermöglicht und auch bei niedrigster Dosierung und mit schwerlöslichen Präparaten eine hohe Dosiergenauigkeit gewährleistet.

Zur Lösung dieser Aufgabe wird gemäß der Erfindung vorgeschlagen, daß das Trägermaterial als saugfähiges, unter Wasseraufnahme quellendes und selbsttätig in einen Faserbrei zerfallendes Faservlies ausgebildet ist und daß der Wirkstoff auf die Oberfläche des Trägermaterials aufgebracht oder in dieses eingebettet ist.

Zur Herstellung der erfindungsgemäßen Tabletten kann als Trägermaterial ein vorgefertigtes Papierfaservlies, insbesondere Zellulosefaservlies, oder ein Textilfaservlies verwendet werden.

Für die Verarbeitung benötigt das Faservlies eine ausreichende Trockenfestigkeit und Formhaltigkeit, damit der Wirkstoff, beispielsweise in Form eines Aufdruckes, auf die Oberfläche aufgebracht werden kann. Andererseits ist es erwünscht, daß das Faservlies und damit die Tablette unter Wasseraufnahme sehr schnell aufquillt und in die einzelnen Fasern zerfällt. Diese beiden, einander scheinbar widersprechenden Eigenschaften können in optimaler Weise aufeinander abgestimmt werden, wenn das Faservlies ein unter Wasseraufnahme quellendes wasserlösliches Bindemittel enthält. Als Bindemittel wird bevorzugt ein hydrophiles Gel, insbesondere Gelatine oder Knochenleim verwendet. Gelatine ist ebenso wie Zellulose als Beimischung in Arzneimitteln toxologisch unbedenklich. Die Zerfalls-geschwindigkeit des Faservlieses kann durch die verwendete Faserstärke und -länge sowie das Quellvermögen des Bindemittels einerseits und durch das verwendete Mengenverhältnis zwischen Fasermaterial und Bindemittel in weiten Grenzen eingestellt werden: Je kürzer und je dicker und damit je weniger verfilzt die Fasern im Faservlies sind und je kleiner der Bindemittelanteil ist, um so schneller zerfällt das Trägermaterial.

Der Wirkstoff kann beispielsweise mit Hilfe einer Mikrodosierpumpe in Form eines Aufdruckes auf das Faservlies aufgebracht werden. Zum Schutz des Trägermaterials kann das Faservlies mit einem porenverschließenden wasserlöslichen oder wasserdurchlässigen Überzug versehen werden, der eine wasserabstoßende Oberfläche aufweisen kann.

Die erfindungsgemäßen Tabletten können über vorzugsweise perforierte, gestanzte oder genutete Abreißkanten zu größeren Einheiten miteinander verbunden sein, beispielsweise als blatt- oder bogenförmiges Gebilde, als Abreißblock oder als auf eine Rolle aufgewickelter Strang. Weiter können die Tabletten aus mehreren, verschiedene Wirkstoffe enthaltenden Vlieslagen bestehen, die beispielsweise durch eine Flächenhaftverbindung, insbesondere eine Klebeverbindung oder eine Riffelstanzung miteinander verbunden sind. Dadurch ist eine genau aufeinander abgestimmte, aber voneinander unabhängige Dosierung der Wirkstoffe ohne mechanische Mischung möglich. Die Mischung tritt erst zum Zeitpunkt der Applikation beim Auflösen der Vlieslagen ein. Grundsätzlich ist es auch möglich, mehrere verschiedene Wirkstoffe in kleinem Abstand voneinander auf ein und dasselbe Vlies aufzutragen, ohne daß eine Lagenbildung und eine vorherige Mischung der Wirkstoffe nötig ist.

Sofern es bei der Einbringung eines Wirkstoffes, beispielsweise eines Düngemittels, oder von Saatkörnern auf eine bestimmte Abstandsverteilung ankommt, ist es vorteilhaft, wenn mehrere den Wirkstoff enthaltende Tabletten in einer Reihe oder in einem Flächenraster im Abstand voneinander auf einer band- oder blattartigen Unterlage angeordnet sind.

0019929

Dabei sollte sich auch die Unterlage allmählich in einer wasserhaltigen Umgebung auflösen oder in dieser verrotten. Die Tabletten sind in diesem Falle vorteilhafterweise mit einer selbsthaftenden, den Wirkstoff tragenden wasserlöslichen Schicht auf der Unterlage befestigt.

Bei einem bevorzugten Verfahren zum Herstellen von Tabletten, bei welchem mindestens ein Wirkstoff mit einer Grund- oder Trägersubstanz verbunden wird, wird der Wirkstoff in kleine, im Abstand voneinander angeordnete Bereiche eines saugfähigen, unter Wasseraufnahme selbsttätig in einen Faserbrei zerfallenden, flächigen Faservlieses ein- oder aufdosiert und das Faservlies vor, während oder nach der Wirkstoffzugabe in kleinere, mindestens einen der Bereiche enthaltende Einheiten aufgetrennt. Der Wirkstoff wird dabei zweckmäßig auf die Oberfläche des Faservlieses aufgetragen oder in das Faservlies injiziert.

Gemäß einer bevorzugten Ausgestaltung wird der Wirkstoff in Form einer Lösung, Suspension oder Schmelze in das Faservlies injiziert oder auf dieses aufgetragen und das Faservlies anschließend getrocknet.

In der Zeichnung sind einige bevorzugte Ausführungsbeispiele der Erfindung in schematischer Weise dargestellt. Es zeigen:

Fig. 1   eine Vliestablette in rechteckiger Form in teilweise aufgebrochener Darstellung;

Fig. 2   eine Vliestablette in runder Form;

Fig. 3   einen Bogen mit einer Mehrzahl von an Abreißkanten
miteinander verbundenen Tabletten;

Fig. 4   eine Rolle mit einer Mehrzahl von an Abreißkanten
miteinander verbundenen Tabletten;

Fig. 5   eine dreilagige Vliestablette in teilweise aufgebrochener Darstellung;

Fig. 6   einen Block mit einer Mehrzahl von an einer Abreißkante miteinander verbundenen Vliestabletten.


Die in der Zeichnung dargestellten Tabletten 1,1', 1'' bestehen aus einem saugfähigen, unter Wasseraufnahme selbsttätig in einen Faserbrei zerfallenden Trägermaterial 8 aus einem Faservlies, in den ein Wirkstoff 2 auf- oder eindosiert ist. Das Faservlies ist bevorzugt als Zellulosefaservlies ausgebildet, das ein unter Wasseraufnahme aufquellendes, wasserlösliches Bindemittel enthält. Das Bindemittel besteht aus Gelatine, Knochenleim oder einem anderen hydrophilen Gel. Die Zerfallsgeschwindigkeit des Vlieses kann durch die verwendete Faserstärke und -länge sowie durch das Quellvermögen des Bindemittels    und durch das verwendete Mengenverhältnis zwischen Fasermaterial und Bindemittel in weiten Grenzen eingestellt werden. Die Vliestabletten lösen

- 8 -

sich typischerweise in einigen Zehntelsekunden bis zu einigen
Sekunden in Wasser auf.

Der chemische, pharmazeutische oder biologische Wirkstoff
wird bevorzugt in flüssiger Form, z.B. in Form einer wäßrigen
oder alkoholischen Lösung, einer Suspension oder einer Schmelze
mit Hilfe eines geeigneten Dosiergeräts, beispielsweise einer
Mikrodosierpumpe, auf die Oberfläche des Faservlieses aufgebracht und dringt aufgrund der Saugfähigkeit etwas in
dieses ein, so daß er nach der Trocknung an den Fasern
haften bleibt. Zur guten Haftung trägt auch das bei flüssiger
Wirkstoffzugabe lokal klebrig werdende Bindemittel im Vlies
bei. Weiter ist auch eine Injektion des flüssigen Wirkstoffs
in das Vliesmaterial oder die Befestigung eines festen Wirkstoffs, beispielsweise von Pflanzensamen, an einer Haftschicht
des Vlieses möglich.

Das vorgefertigte Faservlies liegt üblicherweise in Rollen-
oder Bogenform vor und wird vor, während oder nach der Wirkstoffzugabe in kleinere Einheiten aufgeteilt, die ihrerseits
mehrere über perforierte, gestanzte oder genutete Abreißkanten 3 verbundene Einzeltabletten 1' enthalten können.

In Fig. 1 ist eine viereckige und in Fig. 2 eine runde
Einzeltablette 1 dargestellt, auf deren Oberflächen der
Wirkstoff 2 jeweils in mehreren Bereichen aufgetragen,
beispielsweise in Schriftform aufgedruckt ist. Zum Aufdrucken kann der Wirkstoff mit einem Farbstoff eingefärbt werden.

Bei dem in Fig. 3 gezeigten Ausführungsbeispiel sind mehrere über Abreißkanten 3 und durch Zwischenräume 4 voneinander getrennte Einzeltabletten zu einem blatt- oder bogenförmigen Gebilde 10 miteinander verbunden und im Falle von Fig. 4 auf einer Rolle 11 aufgewickelt. In Fig. 6 ist ein Tablettenstapel in Form eines Abreißblocks 12 dargestellt.

Die in Fig. 5 gezeigte Tablette 1'' besteht aus mehreren Vlieslagen 5,6,7, die unterschiedliche Wirkstoffe 2',2'',2''' enthalten, so daß eine Mischung der Wirkstoffe entbehrlich ist. Dies ist besonders bei nicht mischbaren oder chemisch miteinander reagierenden Präparaten von Vorteil. Die Vlieslagen können durch Flächenhaftverbindungen, beispielsweise durch eine Klebeverbindung oder eine Riffelstanzung miteinander verbunden werden.

Unterschiedliche Wirkstoffe können auch auf einer einlagigen Tablette in voneinander getrennte Oberflächenbereiche aufgebracht werden (vgl. Fig. 1 bis 3 und 6).

0019929

1. Tablette mit einem Trägermaterial und mindestens einem im Trägermaterial enthaltenen Wirkstoff, insbesondere einem Nahrungsmittel, einem pharmazeutischen oder chemischen Präparat, Pflanzen- oder Tiersamen, d a - d u r c h g e k e n n z e i c h n e t , daß das Trägermaterial (8) als saugfähiges, ein unter Wasseraufnahme aufquellendes Bindemittel enthaltendes Faservlies ausgebildet ist, das beim Aufquellen des Bindemittels selbsttätig in einen Faserbrei zerfällt, und daß der Wirkstoff auf die Oberfläche des Trägermaterials aufgebracht oder in dieses eingebettet ist.

2. Tablette nach Anspruch 1, d a d u r c h g e k e n n - z e i c h n e t , daß das Trägermaterial (8) als verhältnismäßig dick- und/oder kurzfaseriges Papierfaservlies, insbesondere Zellstoffaservlies, oder Textilfaservlies ausgebildet ist.

3. Tablette nach Anspruch 1 oder 2, d a d u r c h g e - k e n n z e i c h n e t , daß das Bindemittel ein hydrophiles Gel, insbesondere Gelatine oder Knochenleim ist.

4. Tablette nach einem der Ansprüche 1 bis 3, d a d u r c h g e k e n n z e i c h n e t , daß das Faservlies (8) mit einem den Wirkstoff (2) enthaltenden Aufdruck versehen ist.

0019929

5. Tablette nach einem der Ansprüche 1 bis 4, d a d u r c h
   g e k e n n z e i c h n e t , daß das Faservlies (8) mit
   einem porenverschließenden wasserlöslichen oder wasserdurchlässigen Überzug versehen ist, der vorzugsweise eine
   wasserabstoßende Oberfläche aufweist.

6. Tablette nach einem der Ansprüche 1 bis 5, d a d u r c h
   g e k e n n z e i c h n e t , daß sie mit mindestens einer
   weiteren Tablette (1') über eine vorzugsweise perforierte,
   gestanzte oder genutete Abreißkante (3) verbunden ist.

7. Tablette nach Anspruch 6, d a d u r c h g e k e n n -
   z e i c h n e t , daß sie zusammen mit den mit ihr verbundenen weiteren Tabletten (1') auf eine Rolle (11) oder
   Spule aufgewickelt ist oder einen Abreißblock (12) bildet,
   oder daß sie zusammen mit den weiteren Tabletten (1') als
   blatt- oder bogenförmiges Gebilde (10) ausgebildet ist,
   wobei vorzugsweise zwischen den Tabletten (1') des blatt-
   oder bogenförmigen Gebildes (10) freie, die Abreißkanten
   (3) begrenzende, durch Ausstanzen gebildete Zwischenräume
   (4) vorgesehen sind.

8. Tablette nach einem der Ansprüche 1 bis 7, d a d u r c h
   g e k e n n z e i c h n e t , daß sie aus mindestens
   zwei, verschiedene Wirkstoffe (2', 2'', 2''') enthaltenden
   Vlieslagen (5, 6, 7) besteht, wobei vorzugsweise die Vlieslagen (5, 6, 7) durch eine Flächenhaftverbindung, insbesondere eine Klebeverbindung oder eine Riffelstanzung, miteinander verbunden sind.

9. Tablette nach einem der Ansprüche 1 bis 8, d a d u r c h g e k e n n z e i c h n e t , daß sie in einer Reihe oder einem Flächenraster von im Abstand voneinander auf einer band- oder blattartigen Unterlage angeordneten Tabletten angeordnet ist, wobei sie vorzugsweise mit einer selbsthaftenden, den Wirkstoff tragenden, wasserlöslichen Schicht auf der Unterlage befestigt ist.

10. Verfahren zum Herstellen von Tabletten, bei welchem mindestens ein Wirkstoff mit einer Grund- oder Trägersubstanz verbunden wird, d a d u r c h g e k e n n - z e i c h n e t , daß der Wirkstoff (2; 2', 2'', 2''') in kleine im Abstand voneinander angeordnete Bereiche eines saugfähigen, unter Wasseraufnahme aufquellenden und selbsttätig in einen Faserbrei zerfallenden, flächigen Faservlieses ein- oder aufdosiert und das Faservlies vor, während oder nach der Wirkstoffzugabe in kleinere, mindestens einen der Bereiche enthaltende Einheiten (1, 10, 11) aufgetrennt wird.

11. Verfahren nach Anspruch 10, d a d u r c h g e k e n n - z e i c h n e t , daß der Wirkstoff mit Hilfe einer Mikrodosierpumpe    in Form einer Lösung, Suspension oder
Schmelze in das Faservlies injiziert oder auf dieses aurgetragen oder aufgedruckt und das Faservlies anschließend
getrocknet wird.